**Europäisches Patentamt**

**European Patent Office**

**Office européen des brevets**

(19)

(11) Veröffentlichungsnummer: **0 270 704**
**B1**

(12) **EUROPÄISCHE PATENTSCHRIFT**

(45) Veröffentlichungstag der Patentschrift:
26.04.89

(51) Int. Cl.⁴: **A61B 17/58**, F16B 13/06

(21) Anmeldenummer: **86117360.7**

(22) Anmeldetag: **12.12.86**

(54) **Befestigungselement zur Festlegung einer Osteosyntheseplatte an einem Knochen.**

(43) Veröffentlichungstag der Anmeldung:
**15.06.88 Patentblatt 88/24**

(45) Bekanntmachung des Hinweises auf die Patenterteilung:
**26.04.89 Patentblatt 89/17**

(84) Benannte Vertragsstaaten:
**CH DE ES FR GB LI SE**

(56) Entgegenhaltungen:
**EP-A- 0 022 724**
**EP-A- 0 052 998**
**EP-A- 0 058 744**
**EP-A- 0 124 489**
**WO-A-82/03174**
**FR-A- 2 054 731**
**US-A- 2 381 050**
**US-A- 3 708 883**
**US-A- 3 842 824**
**US-A- 4 013 071**
**US-A- 4 492 226**

(73) Patentinhaber: **AESCULAP AG, Möhringer Strasse 125,
D-7200 Tuttlingen(DE)**

(72) Erfinder: **Lutze, Theodor, Schiller Strasse 2,
D-7201 Balgheim(DE)**

(74) Vertreter: **Hoeger, Stellrecht & Partner,
Uhlandstrasse 14 c, D-7000 Stuttgart 1(DE)**

## Beschreibung

Die Erfindung betrifft ein Befestigungselement zur Festlegung einer mit Bohrungen versehenen Osteosyntheseplatte an einem Knochen.

Zur Verbindung von Knochenteilen werden Osteosyntheseplatten verwendet, die normalerweise mit Schrauben an den miteinander zu verbindenden Knochenteilen befestigt werden. Es ist bekannt, die Osteosyntheseplatten sowie die Schrauben aus speziellem, austenitischem Implantatstahl herzustellen. Dadurch lassen sich die notwendigen Festigkeitseigenschaften erreichen, nachteilig ist jedoch, daß einmal aufgrund der Härte der Platten eine individuelle Anpassung der Platten an das Implantat nur schwer vorgenommen werden kann und daß zum anderen diese Implantate nach einem angemessenen Zeitraum wieder aus dem Körper entfernt werden müssen.

Es sind zwar an sich bereits zur Herstellung von Implantaten verwendbare Substanzen bekannt, die nach einiger Zeit im Körper abgebaut und vom Körper resorbiert werden, so daß eine Entfernung nicht notwendig ist. Derartige Implantatwerkstoffe weisen aber in der Regel nicht dieselbe Festigkeit auf wie die zur Osteosynthese verwendeten Materialien, so daß mit solchen Materialien zwar bisher schon Gefäßclipse oder Stifte zur Reposition von Knorpelfrakturen hergestellt worden sind, diese Materialien waren aber für übliche Osteosyntheseverfahren nicht geeignet. Dies gilt im besonderen Maße für die Befestigungselemente, mit denen Osteosyntheseplatten am Knochen befestigt werden. Bildet man diese in der herkömmlichen Form als Schrauben aus, so gelingt es nicht, diese in den Knochen einzuschrauben, da die Schraubenköpfe beim Einschrauben abreißen.

Es ist Aufgabe der Erfindung, ein Befestigungselement für Osteosyntheseplatten zu schaffen, welches auch bei Verwendung eines weniger festen Materials eine sichere und dauerhafte Fixierung einer Osteosyntheseplatte am Knochen erlaubt.

Diese Aufgabe wird bei einem Befestigungselement der eingangs beschriebenen Art erfindungsgemäß durch die Merkmale des kennzeichnenden Teils des Patentanspruchs 1 gelöst.

Der Dübel hat also äußerlich zwar die Form einer Schraube, so daß er unter Anlage des verbreiterten Kopfes in eine Bohrung der Osteosyntheseplatte eingesetzt werden kann. Der aus der Osteosyntheseplatte in den Knochen hineinragende Teil jedoch trägt kein Außengewinde, sondern er wird im Knochen durch Aufweitung des freien Endes verankert. Diese Aufweitung erfolgt durch einen Spannzapfen, der zum Aufweiten in die Längsbohrung hineingeschoben oder -gezogen wird, so daß der Spannteil die Wand des aufgeweiteten Teiles der Längsbohrung auseinanderzwängt und dadurch den Dübel am freien Ende insgesamt aufweitet.

Es hat sich herausgestellt, daß eine ausgezeichnete Fixierung von Osteosyntheseplatten mit Spannelementen dieser Art möglich ist, so daß zur Herstellung dieser Spannelemente Materialien verwendet werden können, die weniger fest sind als die herkömmlich verwendeten Metallimplantate. Trotzdem läßt sich eine ausreichende Festlegung der Osteosyntheseplatten am Knochen erreichen. Dies führt dazu, daß man die beschriebenen Spannelemente aus resorbierbaren Kunststoffen herstellt, beispielsweise aus Polylactit. Verwendet man derartige Materialien sowohl für die Osteosyntheseplatte selbst als auch für die Befestigungselemente, lösen sich diese Implantate im Körper nach einer bestimmten Zeit auf und werden vom Körper resorbiert, so daß eine Reoperation zur Entnahme der Implantate überflüssig wird.

Um die Aufweitung des freien Endbereiches des Dübels zu erleichtern, ist vorgesehen, daß der Dübel im Bereich der aufgeweiteten Längsbohrung in Längsrichtung geschlitzt ist. Günstig ist auch, wenn der Dübel an seinem aufweitbaren Ende radial vorspringende Rückhalteelemente trägt, die sich in das umgebende Knochengewebe einkrallen können.

Der Schaftteil des Spannzapfens ist so lang, daß er aus dem gegenüberliegenden Ende der Längsbohrung austritt, bevor der Spannteil an der aufgeweiteten Innenwand der Längsbohrung anliegt. Es wird dadurch möglich, den aus dem Kopf des Dübels herausragenden Schaftteil mit einem Werkzeug zu erfassen und unter gleichzeitigem Andrücken des Kopfes an den Knochen so weit aus dem Dübel herauszuziehen, bis der Spannteil des Spannzapfens das freie Ende des Dübels in der gewünschten Weise aufgeweitet hat. Dies kann mit einem geeigneten Werkzeug geschehen.

Zwischen Schaftteil und Spannteil ist eine Sollbruchstelle angeordnet, die durch eine umlaufende Kerbe gebildet ist. Der Spannvorgang des Spannzapfens wird dabei dadurch beendet, daß der Schaftteil von dem fest in den Dübel eingepreßten Spannteil abreißt. Dies führt nicht nur dazu, daß der Spannteil immer mit einer vorbestimmten Kraft in den Dübel eingezogen wird, sondern auch dazu, daß der Spannteil im Inneren des Dübels verbleibt, ohne daß Teile des Spannzapfens aus dem Dübel hervorstehen. Dadurch ist sichergestellt, daß der Spannteil in seiner gespannten Position verbleibt und nicht versehentlich wieder zurückgeschoben werden kann. Außerdem ergeben sich keine über den Dübel selbst hervorstehende Teile, die umliegendes Gewebe verletzen könnten.

Der Spannteil ist an seinem Außenmantel mit in Umfangsrichtung umlaufenden Vorsprüngen versehen und der Dübel weist im Anlagebereich der Innenwand der Längsbohrung am Spannteil mit den Vorsprüngen am Spannteil in Wirkverbindung tretende Vorsprünge auf. Die Vorsprünge können dabei Rippen oder Schultern sein, die in Umfangsrichtung umlaufen, beispielsweise können sowohl der Spannteil als auch die Innenwand der Bohrung mit einer Querriffelung versehen sein. Dadurch ergibt sich zwischen Spannteil und Innenwand der Längsbohrung in Bohrungslängsrichtung eine formschlüssige Verriegelung, die verhindert, daß der Spannteil nach dem Hineinziehen in den Dübel unbeabsichtigt wieder aus diesem herausgleiten kann.

Befestigungselemente der beschriebenen Art sind an sich beispielsweise aus der US-PS 3 708 883 bekannt. Dort werden sie als in den Kiefer einsetzbare Halteelemente verwendet, auf deren aus dem Kiefer hervorstehende Gewindeteile ein künstlicher Zahn aufgeschraubt wird. Diese sehr spezielle Verwendung kann jedoch nicht nahelegen, Spannelemente dieser Art anstelle der herkömmlichen Schrauben zur Festlegung von Osteosyntheseplatten an Knochen einzusetzen.

Die nachfolgende Beschreibung bevorzugter Ausführungsformen der Erfindung dient im Zusammenhang mit der Zeichnung der näheren Erläuterung. Es zeigen:

Figur 1: eine Seitenansicht eines teilweise aufgebrochen dargestellten Oberschenkelknochens mit einer Osteosyntheseplatte, die mittels erfindungsgemäßer Befestigungselemente an den Knochenfragmenten befestigt ist;

Figur 2: eine Knochenverbindung bei zwei plattenförmigen Knochenelementen;

Figur 3: eine Teilschnittansicht eines Dübels eines Befestigungselementes;

Figur 4: eine Seitenansicht eines Spannzapfens eines Befestigungselementes;

Figur 5: eine Schnittansicht eines Dübels mit eingesetztem Spannzapfen vor dem Spannen;

Figur 6: eine Ansicht ähnlich Figur 5 nach dem Spannen des Spannzapfens mit abgerissenem Schaftteil und

Figur 7: eine vergrößerte Detailansicht entsprechend Ausschnitt A in Figur 6.

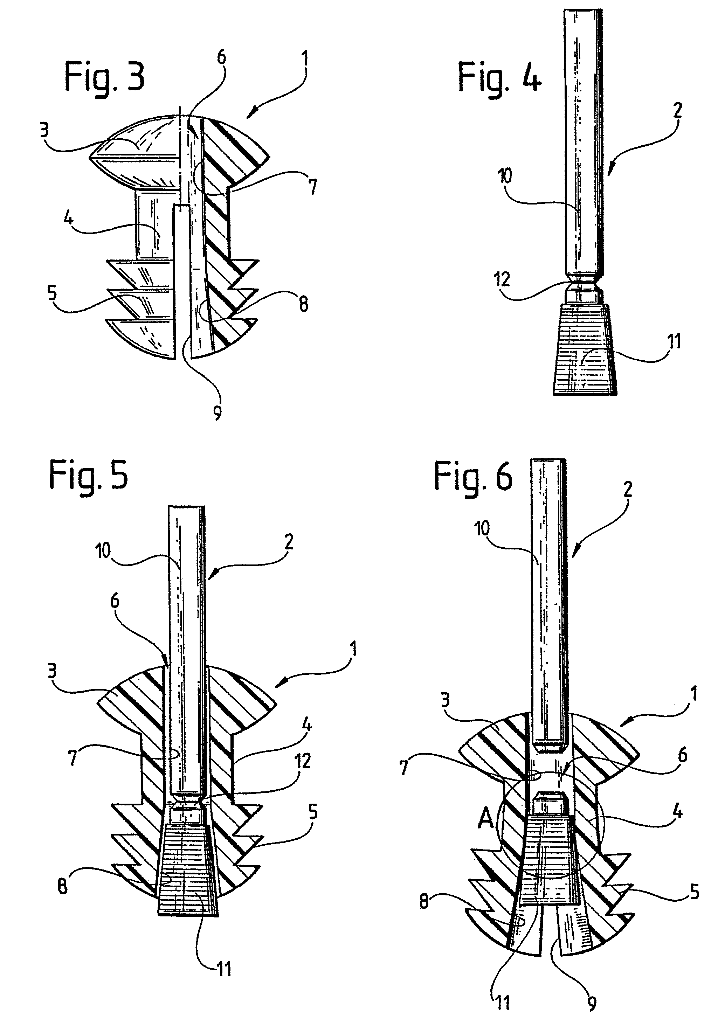

Das in den Figuren 3 bis 6 im Detail dargestellte Befestigungselement umfaßt einen Dübel 1 (Figur 3) und einen Spannzapfen 2 (Figur 4). Der Dübel 1 weist einen linsenförmigen Kopf 3 auf, wie er an sich von Schrauben bekannt ist. An diesen schließt sich ein Schaft 4 an, der jedoch im Gegensatz zu Knochenschrauben kein Außengewinde trägt, sondern im Bereich seines freien Endes sägezahnartig nach außen abstehende Umfangsrippen 5, die als Rückhalteelemente wirken. Ei ne Längsbohrung 6 durchsetzt den gesamten Dübel 1. Diese ist in dem dem Kopf 3 benachbarten Bereich 7 zylindrisch ausgebildet, in dem dem Kopf abgewandten Bereich des Dübels 1 weitet sich die Längsbohrung 6 in Richtung zum Ende des Dübels 1 auf, so daß ein sich konisch erweiternder Spannbereich 8 entsteht. Der Dübel 1 ist bis an den Ansatzpunkt des Kopfes 3 mit zwei einander diametral gegenüberliegenden Längsschlitzen 9 versehen, die sich bis in die Längsbohrung 6 hinein erstrecken.

Der Spannzapfen 2 umfaßt ein zylindrisches Schaftteil 10, dessen Außendurchmesser so gewählt ist, daß er kleiner oder allenfalls gleich dem Innendurchmesser des zylindrischen Bereiches 7 der Längsbohrung 6 ist. An diesen zylindrischen Schaftteil 10 schließt sich ein sich konisch aufweitender Spannteil 11 an, dessen Konizität der des Spannbereiches 8 der Längsbohrung 6 entspricht, solange die Längsbohrung 6 in diesem Bereich noch nicht aufgeweitet ist. Im Übergangsbereich zwischen zylindrischem Schaftteil 10 und konischem Spannteil 11 ist der Schaftteil 10 durch eine umlaufende Kerbe 12 geschwächt, so daß in diesem Bereich eine Sollbruchstelle entsteht.

Sowohl der Dübel 1 als auch der Spannzapfen 2 bestehen vorzugsweise aus einem resorbierbaren Material, insbesondere aus Polylactit. Es können aber auch andere Kunststoffmaterialien verwendet werden.

Die beschriebenen Befestigungselemente werden zur Befestigung von Osteosyntheseplatten 13 an einem Knochen 14 verwendet. Die Osteosyntheseplatten 13 können dabei grundsätzlich aus beliebigem Material bestehen, vorzugsweise bestehen auch diese Osteosyntheseplatten aus einem im Körper resorbierbaren Material, beispielsweise aus Polylactit. Die Osteosyntheseplatten können alle herkömmlichen Formen einnehmen, dabei können bereits vorgeformte Osteosyntheseplatten oder solche verwendet werden, die erst bei der Operation aus einer Platte geschnitten, durch Sägen oder Schneiden geformt, gebohrt und gegebenenfalls durch Wärmeeinwirkung an die Kontur des Knochens angepaßt werden.

Zur Befestigung einer solchen Osteosyntheseplatte an einem Knochen wird ein Dübel 1, in den ein Spannzapfen 2 in der aus Figur 5 ersichtlichen Weise eingesetzt ist, durch eine Öffnung 15 in der Osteosyntheseplatte 13 hindurchgestreckt, wie dies von Knochenschrauben an sich bekannt ist. Der Dübel des Befestigungselementes wird dann in eine entsprechende Bohrung im Knochen hineingesteckt, bis die Osteosyntheseplatte 13 fest an dem Knochen anliegt.

Der Spannzapfen 2 ist so lang ausgebildet, daß der in den Dübel eingesteckte, aber noch nicht gespannte Spannzapfen 2 aus dem Kopf 3 des Dübels 1 hervorsteht (Figur 5). Zur Festlegung des Dübels im Knochen wird der aus dem Kopf 3 hervorstehende Schaftteil 10 mittels eines geeigneten, zangenförmigen Werkzeuges erfaßt und aus dem Dübel herausgezogen, wobei gleichzeitig der Kopf des Dübels kräftig gegen die Osteosyntheseplatte gedrückt wird, die dabei gleichzeitig fest an den Knochen angelegt wird. Dabei wird der konische Schaftteil 10 in den konisch aufgeweiteten Spannbereich 8 in der Längsbohrung 6 hineingezogen und weitet dadurch den freien Endteil des Dübels 1 auf. Diese Aufweitung wird durch die Längsschlitze 9 erleichtert (Figur 6). Dabei krallen sich die Umfangsrippen 5 in das umgebende Spongiosagewebe des Knochens und fixieren den Dübel sicher im Knochen.

Beim Überschreiten einer bestimmten Zugkraft reißt der Schaftteil 10 im Bereich der als Sollbruchstelle wirkenden Kerbe 12 ab, so daß der Schaftteil aus dem Dübel 1 herausgezogen werden kann (Figur 6). Im Dübel verbleibt dann nur der Spannteil 11, der dafür sorgt, daß der Dübel in der aufgespreizten und im Knochen fixierten Stellung festgelegt ist.

Der Spannteil 11 trägt auf seinem Außenmantel 16 eine horizontal umlaufende Riffelung, so daß dort mehrere parallel zueinander umlaufende, rippenförmige Vorsprünge 17 gebildet werden. In gleicher Weise ist die Innenwand der Längsbohrung 6 geriffelt, so daß auch diese horizontal umlaufende Vorsprünge 18 trägt. Die Vorsprünge 17 und 18 sind dabei so ausgebildet, daß sie komplementär formschlüssig ineinandergreifen, wenn der Spannteil 11 in die Längsbohrung 6 hineingezogen wird. Dadurch ergibt sich eine axiale Fixierung des Spannteiles 11 in der Längsbohrung 6, das heißt ein unbeabsichtigtes Verschieben des Spannteils 11 aus seiner Spannposition heraus ist nicht mehr möglich.

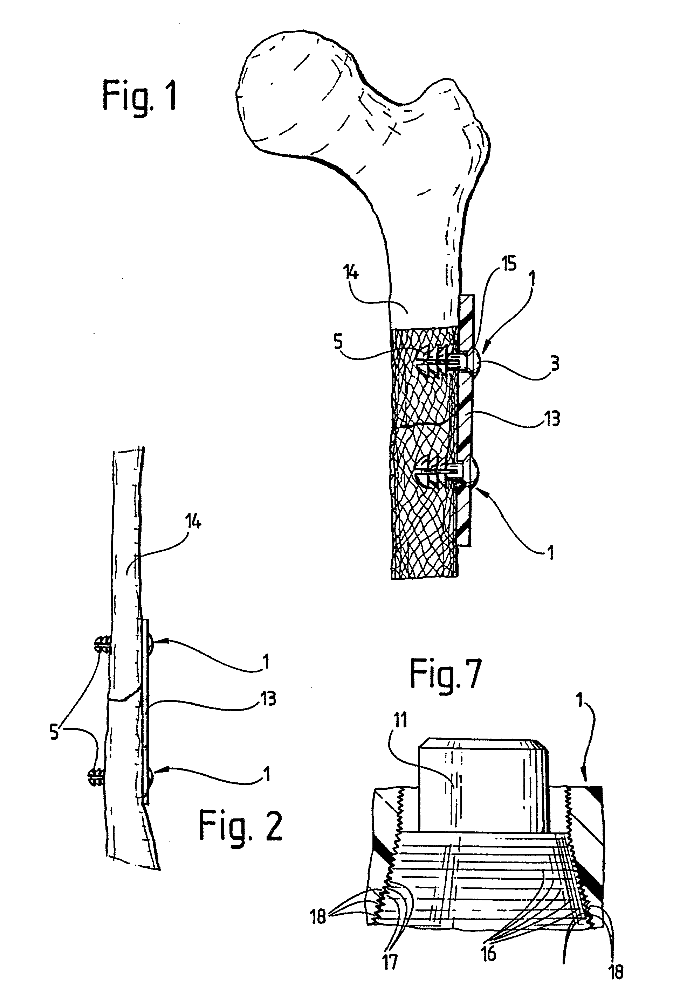

Bei dem Ausführungsbeispiel der Figur 1 befindet sich der gesamte mit Umfangsrippen 5 versehene Teil des Dübels im Inneren des Knochens. Bei dem Ausführungsbeispiel der Figur 2, bei dem relativ dünne, plattenförmige Knochenteile miteinander verbunden werden sollen, beispielsweise Teile eines Schädel- oder Gesichtsknochens, treten die mit Umfangsrippen 5 versehenen Teile des Dübels 1 auf der der Osteosyntheseplatte gegenüberliegenden Seite aus dem Knochen hervor. In diesem aus dem Knochen heraustretenden Teil wird der Dübel 1 aufgespreizt, so daß auch auf diese Weise eine sichere Festlegung der Dübel im Knochen möglich ist.

Die beschriebenen Befestigungselemente halten die Osteosyntheseplatte im Knochen so sicher, daß Materialien verwendet werden können, die geringere Festigkeitswerte als herkömmliche Implantatwerkstoffe aufweisen. Dies ist einmal dann von Vorteil, wenn resorbierbare Materialien verwendet werden, zum anderen können auch Kunststoffmaterialien verwendet werden, die beispielsweise in Computertomogrammen oder in Kernresonanztomogrammen keine unerwünschten Störungen hervorrufen.

## Patentansprüche

1. Befestigungselement zur Festlegung einer mit Bohrungen versehenen Osteosyntheseplatte an einem Knochen, dadurch gekennzeichnet, daß es einen Dübel (1) mit einem verbreiterten Kopf (3) und einer durchgehenden, sich an ihrem dem Kopf (3) abgewandten Ende aufweitenden Längsbohrung (6) sowie einen Spannzapfen (2) aufweist, der einen ersten, durch die Längsbohrung (6) hindurchpassenden Schaftteil (10) und einen sich aufweitenden zweiten Spannteil (11) umfaßt, der beim Einschieben des Spannzapfens (2) in die Längsbohrung (6) an der Wand des aufgeweiteten Teils (8) der Längsbohrung (6) anliegt und dadurch den Dübel (1) in diesem Endbereich aufweitet, daß der Dübel (1) im Bereich der aufgeweiteten Längsbohrung (6) in Längsrichtung geschlitzt ist, daß der Schaftteil (10) des Spannzapfens (2) so lang ist, daß er aus dem gegenüberliegenden Ende der Längsbohrung (6) austritt, bevor der Spannteil (11) an der aufgeweiteten Innenwand der Längsbohrung (6) anliegt, und daß zwischen Schaftteil (10) und Spannteil (11) eine Sollbruchstelle angeordnet ist, die durch eine umlaufende Kerbe (12) gebildet ist, daß der Spannteil (11) an seinem Außenmantel (16) mit in Umfangsrichtung umlaufenden Vorsprüngen (17) versehen ist, während der Dübel (1) im Anlagebereich der Innenwand der Längsbohrung (6) am Spannteil (11) mit den Vorsprüngen (17) am Spannteil (11) in Wirkverbindung tretende Vorsprünge aufweist, und daß der Dübel (1) und der Spannzapfen (2) und/oder die Osteosyntheseplatte (13) aus einem vom Körper resorbierbaren Werkstoff bestehen.

2. Befestigungselement nach Anspruch 1, dadurch gekennzeichnet, daß der Dübel (1) an seinem aufweitbaren Ende radial vorspringende Rückhalteelemente (5) trägt.

## Claims

1. Attachment element to anchor on a bone an osteo-synthesis-plate which has bores, characterized in that it has a peg (1) with a broadened head (3) and a through-going, longitudinal bore (6) which widens at its end facing away from the head (3) and a tensioning pin (2) which comprises a first shank part (10) which passes through the longitudinal bore (6) and a second tensioning part (11) which when the tensioning pin (2) is pushed into the longitudinal bore (6), rests against the wall of the widened part (8) of the longitudinal bore (6) and by this means widens the peg (1) in this end area; in that the peg (1) is split in the longitudinal direction in the area of the widened longitudinal bore (6); in that the shank part (10) of the tensioning pin (2) is of such a length that it emerges from the opposite end of the longitudinal bore (6) before the tensioning part (11) rests against the widened inside wall of the longitudinal bore (6); and in that between the shank part (10) and the tensioning part (11) a predetermined breaking point is arranged, this being formed by means of an encircling notch (12); in that the tensioning part (11) has projections (17) on its outer jacket, these projections (17) encircling the latter in the circumferential direction, while in the area where the inner wall of the longitudinal bore (6) is in contact on the tensioning part, the peg (1) has projections which enter into an operative connection with the projections (17) on the tensioning part (11); and in that the peg (1) and the tensioning pin (2) and/or the osteo-synthesis-plate (13) are composed of a material which can be reabsorbed by the body.

2. Attachment element according to claim 1, characterized in that the peg (1) bears radially projecting retaining elements (5) on its end which can be widened.

## Revendications

1. Elément de fixation destiné à fixer à un os une plaque d'ostéosynthèse percée de trous, caractérisé en ce qu'il présente une cheville (1) munie d'une tête élargie (3) et d'une lumière longitudinale (6) traversante, qui s'évase à son extrémité la plus éloignée de la tête (3), ainsi qu'un tenon de serrage (2) qui comprend lui-même une première partie ou partie tige (10) qui s'étend à travers la lumière longitudinale (6), et une deuxième partie ou partie de serrage (11) qui s'élargit et qui, lorsque la partie de serrage (2) est enfoncée dans la lumière longitudinale (3), s'appuie contre la paroi de la partie évasée (8) de la lumière longitudinale (6) et, de cette façon, dilate la cheville (1) dans cette région d'extrémité, en ce que la cheville (1) est fendue dans la direction longitudinale dans la région de la lumière longitudinale évasée (6), en ce que la partie tige (10) du tenon de serrage (2) est d'une longueur telle qu'elle émerge de l'extrémité opposée de la lumière longitudinale (6) avant que la partie de serrage (11) ne soit en appui contre la paroi interne évasée de la lumière longitudinale (6) et en ce que, entre la partie tige (10) et la partie de serrage (11), est interposée une amorce de rupture qui est formée par une entaille circonférentielle (12), en ce que la partie de serrage (11) est munie sur sa surface latérale externe (16) de saillies (17) qui s'étendent dans la direction circonférentielle, tandis que la cheville (1) présente, dans la région où la paroi interne de la lumière longitudinale (6) s'applique contre la partie de serrage (11), des saillies qui coopèrent avec les saillies (17) de la partie de serrage (11), et en ce que la cheville (1) et le tenon de serrage (2) et/ou la plaque d'ostéosynthèse (3) sont faits d'une matière qui peut être résorbée par le corps.

2. Elément de fixation selon la revendication 1, caractérisé en ce que la cheville (1) porte des éléments de retenue (5) en saillie radiale sur son extrémité dilatable.

Fig. 1

14
5
15
1
3
13
1

14
5
1
13
1

Fig. 2

Fig. 7

11
1
18
17
16
18

EP 0 270 704 B1

Fig. 3

Fig. 4

Fig. 5

Fig. 6